# EUROPEAN PATENT SPECIFICATION

(11) **EP 2 796 154 B1**
(45) Date of publication and mention of the grant of the patent: **27.09.2017**
(21) Application number: 12859706.9
(22) Date of filing: 20.12.2012
(51) Int. Cl.: A61L 2/00, A61L 2/16, A61L 2/18, A61L 2/20

(54) **DISINFECTING APPLIANCE**
DESINFEKTIONSVORRICHTUNG
APPAREIL DE DÉSINFECTION

(30) Priority: 22.12.2011 ES 201101350
(43) Date of publication of application: 29.10.2014
(73) Proprietor: Saniers S.L., 31195 Berriozar (ES)
(72) Inventor: MUNIAIN LATASA, Javier Antonio, E-31008 Pamplona (ES)
(74) Representative: Cabinet Plasseraud
(86) International application number: PCT/ES2012/070889
(87) International publication number: WO 2013/093162

(56) References cited:
- EP-A1- 1 277 480
- WO-A1-94/26432
- WO-A1-2008/020770
- WO-A1-2011/158957
- DE-A1- 3 517 199
- ES-A1- 2 161 185
- FR-A1- 2 615 738
- KR-A- 20080 021 299
- KR-A- 20110 105 972
- KR-A- 20110 114 205
- US-A- 4 517 159
- US-A1- 2005 163 678
- US-A1- 2005 220 665

## Description

### Field of the Art

The present invention relates to an appliance intended for disinfecting objects used daily, such as baby bottles and the nipples thereof, contact lenses, toothbrushes, etc., in order to prevent the risk of infections as a result of using said objects.

### State of the Art

Research has shown that a problem arises in home environments, work environments, etc., in relation to the use of objects which, due to their nature and use, are susceptible to incorporating harmful microbes which can cause infectious contaminations, such that since there are no suitable means for disinfecting said objects, they are normally used assuming a high contamination risk.

There are disinfection methods today that solve the problem in relation to specific objects, but in a manner that is particularly specific to certain objects and not in a general manner; furthermore the use of said methods is uncomfortable and rather impractical, so they are frequently rejected by user, the contamination risk problem continuing to exist almost in its entirety.

Studies have led to the conclusion that the solution for generalizing the disinfection of objects at risk is determined by the particular shapes of the different types of objects and the difficulty of being able to reach in each case the critical points where hard-to-eliminate organisms settle, it being necessary in some cases to use very aggressive methods which are damaging and therefore inapplicable for some objects, in addition to the practical difficulties of using said methods when they are applicable.

Solutions based on ozone disinfection are known in which the objects to be disinfected are placed in places where ozone is discharged on the objects to be disinfected, or where an ozonated environment is created and it surrounds and penetrates the objects, the solutions of this type known until now being very complex in functional equipment for creating ozone and applying it effectively for disinfecting objects, in addition to comprising a rather impractical setup for use and with significant limitations for placing the objects to be disinfected. Related examples of the prior art can be found:
EP 1 277 480 A1 - "Apparatus for Disinfecting Objects"
US 2005/163678 A1 - "Apparatus and Methods for Therapeutic Use of Ozone"
KR 2011 0114205 A - "Vegetable and Fruit Automatic Washer"
KR 2008 0021299 A - "Vegetable and Fruit Automatic Washer"
WO 94/26432 A1 - "An Endoscope Cleaner/Sterilizer"
FR 2 615 738 A1 - "Process for Decontamination and Cleaning of Ocular Prostheses, in Particular Contact Lenses, and Apparatus for Practicing this Process"

### Object of the Invention

The present invention proposes a disinfecting appliance offering very advantageous constructive and functional features for the disinfecting function, having a unit formed by different independent parts coupled together by means of a simple and practical assembly.

This appliance object of the invention is defined in claim 1 and is formed by three independent parts that can be easily coupled and decoupled, comprising a body housing the operating equipment, a container for the inclusion of the objects to be disinfected in a liquid medium such as water and a lid for closing the container, in which lid there are incorporated means for locking the assembly between the various parts in an integral unit, with actuation means for releasing said assembly.

The means for locking the assembly are formed by a mechanism including two latches for locking the lid and the housing container for housing the objects to be disinfected, and another latch for locking the lid and the body housing the operating equipment, having a button which allows unlocking all of said latches at the same time, and another button which allows individually unlocking the latch locking the lid and the body housing the operating equipment.

The body housing the operating equipment comprises a front closure cover which is fixed by screws, said cover incorporating a formation with side guides, to which there is coupled another plate provided with reciprocal coupling guides, whereas the housing container for housing the objects to be disinfected is in turn coupled on this second plate by means of insertion between reciprocal guides.

Said second plate is securely attached to the lid of the housing container for housing the objects to be disinfected, so the three component parts of the appliance are coupleable and decoupleable by sliding between the mentioned guides, in combination with the locking and unlocking by means of the mechanism incorporated in the lid.

The two plates for coupling between the parts of the appliance also include a tubular socket coupling, which connects and disconnects on its own when the coupling and decoupling of said plates is performed, that tubular socket coupling determining a communication link between a conduit coming from the operating equipment and a tubular pipe communicated with a series of outlets projecting through the plate on which the housing container for housing the objects to be disinfected is coupled.

The mentioned outlets of the tubular pipe are located facing the inside of the housing container for housing the objects to be disinfected in the assembled arrangement of the unit, changing the shape and distribution of said outlets depending on the objects of application for which the appliance is intended. According to the invention the appliance is envisaged for receptacles such, as receptacles of baby bottles or the like, in which the outlets are placed projecting with a specific downward inclination, incorporating a tubular prolongation with elements provided with elastic radial arms. The receptacles to be disinfected can be easily secured onto the tubular prolongations of the outlets, being located, as a result of the inclined position, in a suitable arrangement so that said receptacles are progressively filled completely with the liquid contained in the housing container, so the liquid contacts the entire surface of the receptacles of application, the disinfecting effect being produced on the receptacles being fully effective, the discharge outlets of the disinfecting medium being located inside said receptacles of application further contributing to said disinfecting effect. The outlets are also envisaged to be distributed such that they are submerged progressively, one after another, when introduced into the housing container, thus preventing the combined buoyancy effect of all of said outlets, to facilitate the introduction as well as to prevent overflowing the housing container containing the amount of liquid necessary so that all the receptacles to be disinfected are completely covered.

The operating equipment comprises an air-driving motor pump, an ozone generator, through which the motor pump drives the air, and an on-off solenoid valve for opening and closing the passage towards the tubular pipe which runs into the discharge outlets in the housing container, an electrical power supply and control system being arranged in combination with said unit.

The power system comprises a power supply source with transformation and rectification means, providing a power outlet for the operation of the means for producing ozone and a low-voltage outlet through a stabilizer for the operation of a microswitch controlling the entire functional behavior, showing the operating states by means of indicators.

The ozone generator is operated by means of a multistage voltage doubler, whereby a very high application voltage is obtained, said doubler being provided with high impedance resistances at the outlet, whereby a rapid discharge of the electrical voltage is achieved during disconnection to interrupt the operation to prevent parasitic charges from being able to affect users.

Power is supplied to the operating equipment by means of a square wave having constant pulses with variable gaps that can be regulated, allowing determining automatic activation and deactivation in operating cycles for ozone production in the manner suited to the application. Pulse intensity can also be regulated, thereby allowing changing the revolutions of the motor pump without altering electrical frequency.

Operation occurs without electromagnetic influences, with complete low voltage safety, which fully allows the application in home environment, complying with the specifications established in this regard.

Furthermore, a safety switch is envisaged in the coupling for closing the component parts of the appliance, through which switch the power supply to the operating equipment is disconnected while the entire unit is not completely closed, thus preventing risks of possible accidents.

As a result of the foregoing, this appliance object of the invention certainly has very advantageous features, acquiring its own identity and preferred character for its intended function.

### Description of the Drawings

Figure 1 shows an exploded perspective view of a practical embodiment of the appliance of the invention.

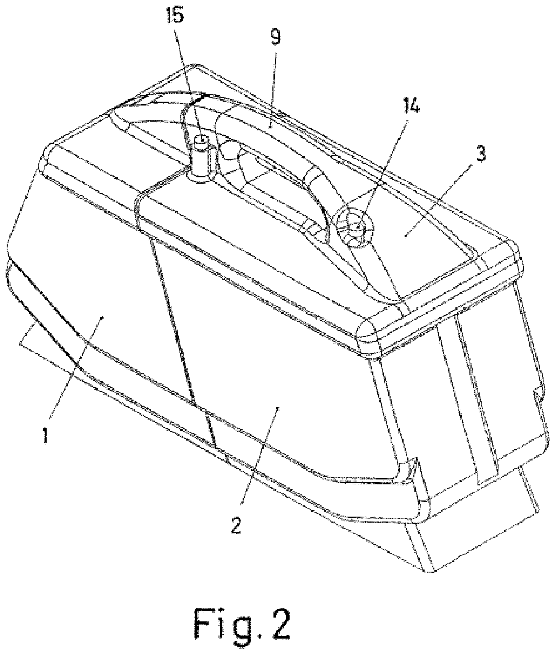

Figure 2 is a perspective view of the assembled appliance.
Figure 3 is a perspective view of the appliance without the upper casing of the lid of the housing container for housing the objects to be disinfected.

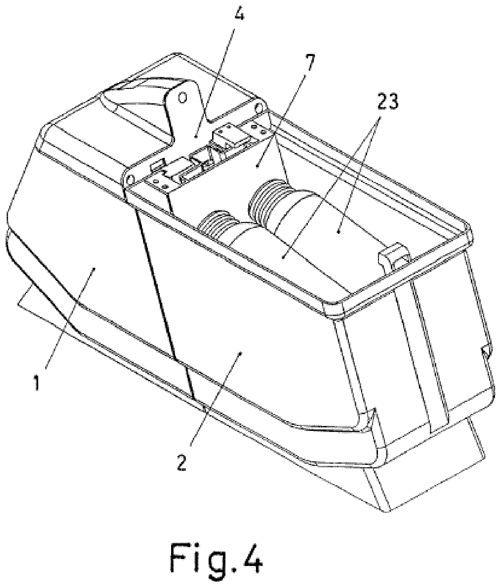

Figure 4 is a perspective view of the appliance without said lid of the housing container for housing the objects to be disinfected.
Figure 5 is a perspective view of the appliance without the receptacle for housing the objects to be disinfected.
Figure 6 is a perspective view of the body housing the operating equipment, the plate in which the ozone discharge outlets are located being arranged facing said body.
Figure 7 is a perspective view seen from the rear part of the mentioned plate incorporating the ozone discharge outlets.
Figure 8 is a side view of the coupling between the closure plate for closing the body housing the operating equipment and the plate incorporating the ozone discharge outlets.
Figure 9 is an enlarged detail of an area of the preceding figure.
Figure 10 is a perspective view of the body housing the operating equipment, without the front closure plate for closing said body.
Figure 11 is a perspective view seen from the outer part of said body housing the operating equipment.
Figure 12 is a transparent side view of the appliance of the invention.

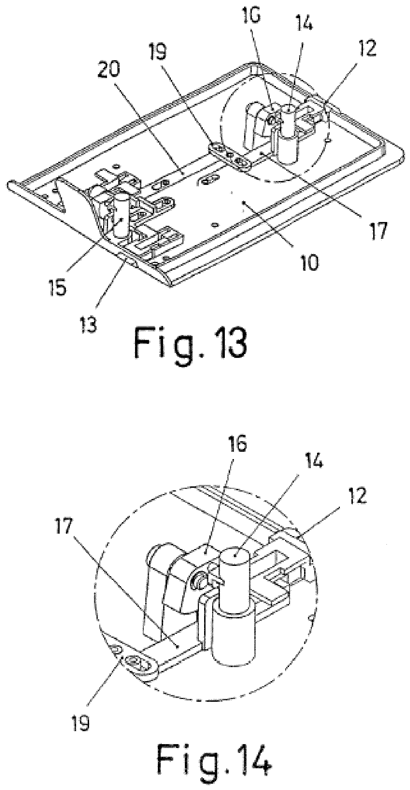

Figure 13 is a perspective view of the locking mechanism for locking the closure of the appliance.
Figure 14 is an enlarged detail of an area of the preceding figure.
Figure 15 is a plan view of the mentioned locking mechanism for locking the closure of the appliance.
Figure 16 is a side view of an enlarged detail of an actuator of the mentioned locking mechanism for locking the closure of the appliance.
Figure 17 is an exploded perspective view of the air-driving motor pump of the appliance.
Figure 18 is a diagram of the power supply source of the electrical equipment of the appliance.
Figure 19 is a diagram of the electrical layout of the control microswitch.

### Detailed Description of the Invention

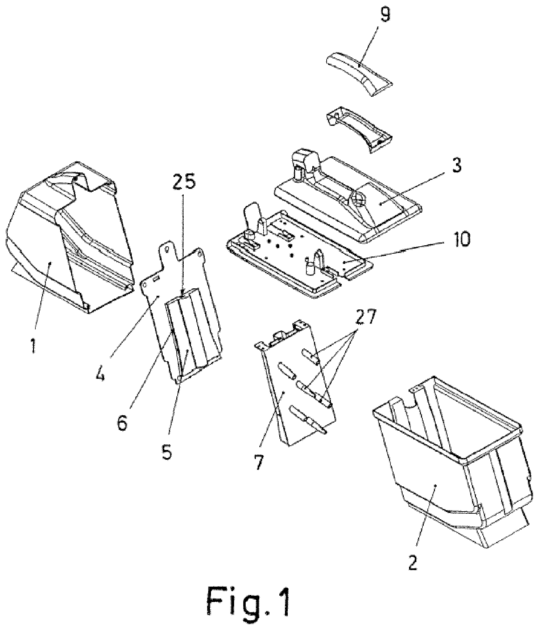

According to the unit depicted in Figure 1, the disinfecting appliance object of the invention comprises a body (1) which houses the operating equipment, a container (2) intended for housing the objects to be disinfected in a liquid medium such as water, and a lid (3) for closing the mentioned container (2).

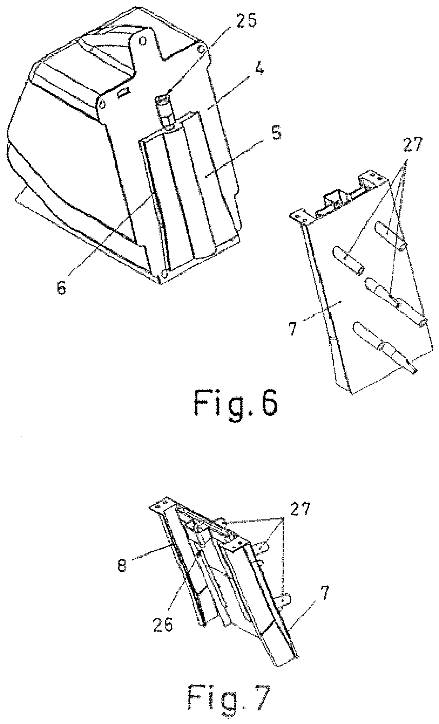

There is placed on the front of the body (1) a closure plate (4) fixed to the mentioned body (1) with screws, as shown in Figure 6, said plate (4) determining a formation (5) provided on the sides with guides (6).

There is fixed to the lid (3) another plate (7) which determines guides (8) (see Figure 7) reciprocal with the guides (6) of the formation (5) of the plate (4), such that both plates (4 and 7) are coupled to one another by insertion fitting between their guides (6 and 8), said coupling guides (6 and 8) progressively increasing in width towards the lower part to facilitate the insertion of the coupling assembly between them.

In addition, the plate (7) is coupled to the housing container (2) for housing the objects to be disinfected by means of a corresponding fitting in order to secure this container (2) to the appliance, the lid (3) establishing closure being tightly fitted on the upper part thereof.

The lid (3) comprises a handle (9) for manipulating and handling the appliance during use, whereas said lid (3) includes a plate (10) on the lower part, whereby the closure contour on the container (2) is established.

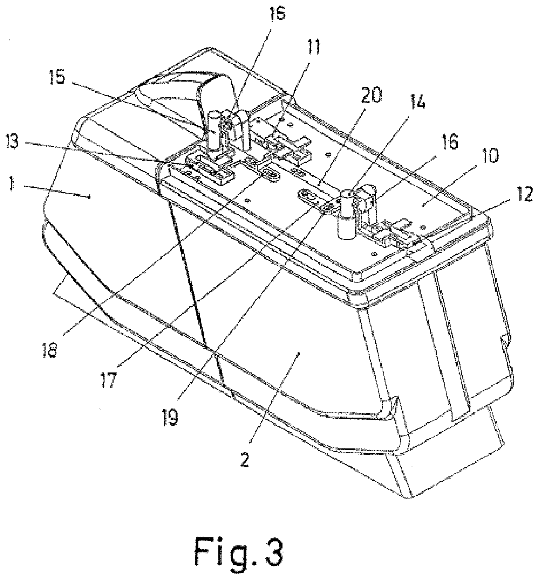

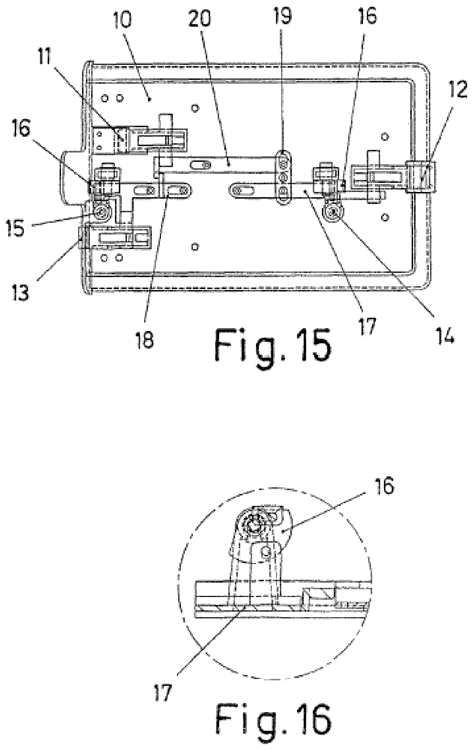

A locking mechanism for locking the closure is incorporated in the mentioned lower plate (10) of the lid (3), said mechanism (see Figures 3, 15 and 16) comprising three latches (11, 12 and 13), retention locking with respect to the container (2) being established by means of two of said latches (11 and 12), whereas retention locking on the plate (4) fixed to the body (1) which houses the operating equipment being established with the other latch (13), the mentioned latches (11, 12 and 13) being actuated by springs that push towards the respective locking positions.

Said mechanism has two push-buttons (14 and 15) which actuate respective rocker arms (16) actuating respective connecting rods (17 and 18), one of which (18) directly actuates the latch (13), whereas the other connecting rod (17), on one hand, directly actuates the latch (12) and, on the other hand, a lever (19), which in turn actuates another connecting rod (20) actuating the latch (11) and the connecting rod (18) actuating the latch (13).

Therefore, the latch (13) can be unlocked by actuating the button (15), allowing decoupling the unit comprised of the lid (3) and the container (2) from the body (1), such that after said decoupling, the latches (11 and 12) can be unlocked by actuating the button (14) to separate the lid (3) with the plate (7) from the container (2).

If the button (14) is actuated when the entire unit is coupled together, the three latches (11, 12 and 13) are thereby unlocked, which allows separating the lid (3) with the plate (7) from the body (1) and from the container (2) at the same time.

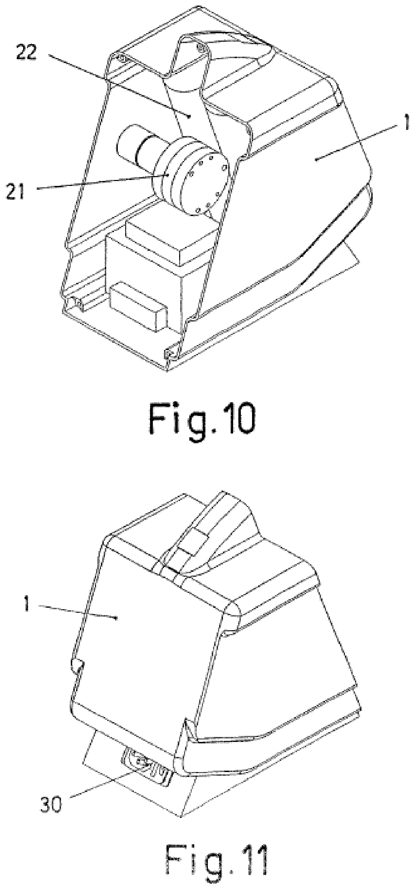

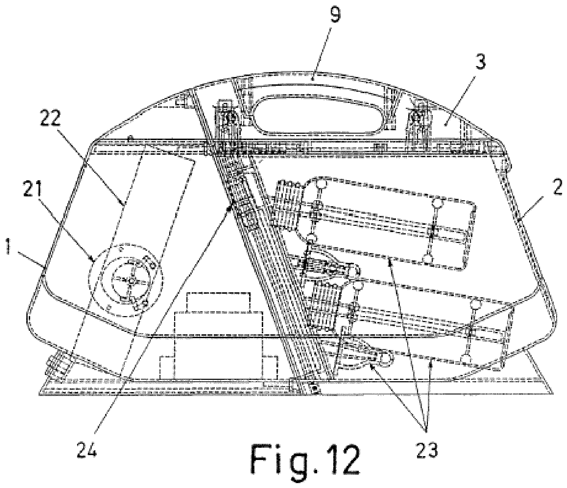

The operating equipment housed in the body (1) comprises (Figures 10 and 12) an air-driving motor pump (21) which takes in air through a filter and drives it through an ozone generator (22) from which the ozonated air is carried through a conduit to be discharged into the container (2) housing the objects (23) to be disinfected, there being included in the ozonated air circulation conduit a solenoid valve (not depicted) automatically opening and closing the passage through said conduit depending on the operating states. Preferably, but without it being limiting, the arrangement of two ozone generators (22) in parallel is envisaged for assuring ozone production when using the appliance.

There is included between the plates (4 and 7) a tubular socket coupling (24) formed by a mouth (25) in which the ozonated air circulation conduit coming from the ozone generators (22) ends up on the plate (4), and by a tubular end (26) incorporated in the plate (7) in an arrangement such that as said plate (7) is coupled to the plate (4) when incorporating the assembly, the mentioned tubular end (26) is inserted into the mouth (25), an automatic connection of said tubular end (26) with the ozone circulation conduit thus being established, when the entire appliance is coupled, whereas when decoupling parts of the appliance the socket is readily disconnected, allowing decoupling.

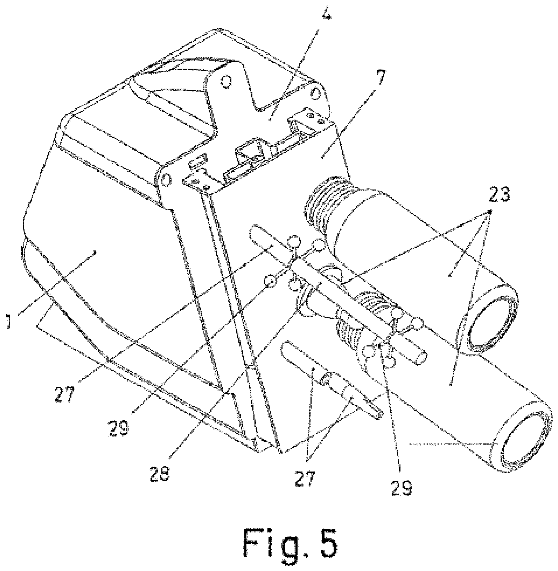

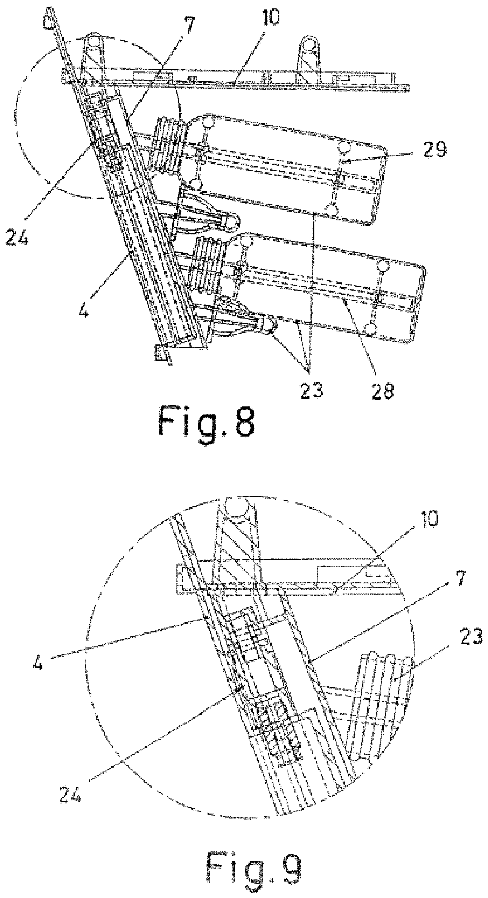

The tubular end (26) in the plate (7) is attached to a conduit projecting through said plate (7) in a series of outlets (27) which are distributed on the mentioned plate (7) depending on the specific application of the appliance. According to the invention which is particularly envisaged for disinfecting objects (23) such as baby bottles and the nipples thereof, the outlets (27) are placed downwardly inclined on the plate (7), with a specific angle of inclination with respect to the horizontal, as seen in Figures 5, 6, 8 and 12.

Said outlets (27) are also established according to a distribution such that the objects (23) to be disinfected in the form of receptacles are distributed in a staggered manner from bottom to top when being arranged on the corresponding outlets (27).

This is because the objects (23) to be disinfected are housed inside the container (2) of the appliance in a liquid medium contained in said container (2), which in the case of objects (23) in the form of receptacles causes a buoyancy that tends to move the unit bearing the objects (23) upwards until said objects are filled with the liquid medium, such that with the mentioned distribution the objects (23) in the form of receptacles fill up successively as they move upwards, thus facilitating introduction in the liquid medium against buoyancy.

For the disinfection to be effective, the liquid medium which receives and propagates the ozone discharged through the outlets (27) must completely cover the objects (23), such that the container (2) must contain a specific amount of said liquid medium so that it completely covers the objects (23) when they are introduced in the mentioned container (2). For such purpose, the staggered vertical distribution of the outlets (27) for incorporating the objects (23) in the form of receptacles is also of fundamental importance so that the liquid medium is progressively introduced in the receptacles (23) to be disinfected, without flooding the housing container (2).

On the other hand, the inclination of the outlets (27) favors fitting the objects (23) to be disinfected in the form of receptacles thereon when the objects (23) are introduced in the liquid medium of the container (2), since the buoyancy effect causes the objects (23) in the form of receptacles to have the tendency to move in the direction in which they are fitted on the outlets (27), without coming off them.

The inclined position also favors displacing air from inside the objects (23) in the form of receptacles as the liquid medium gradually enters said receptacles, such that said objects (23) in the form of receptacles are completely filled with the liquid medium, without there being any air bubbles therein, which determines complete contact of the liquid medium with the surface of the mentioned objects (23) in the form of receptacles for suitable disinfection thereof.

The outlets (27) intended for arranging objects (23) to be disinfected in the form of receptacles are complemented with tubular prolongations (28), between which elements (29) having elastic radial arms are also incorporated, such that the mentioned elements (29) having elastic radial arms allow easily incorporating the objects (23) in the form of receptacles on the outlets (27), said elements (29) passing through the mouth thereof, securing the corresponding object (23) once it is inside by means of their elastic radial arms.

The prolongations (28) allow ozone to be discharged into the lower part of the objects (23) to be disinfected in the form of receptacles, the inside of said objects (23) in the form of receptacles thereby being effectively disinfected with complete efficacy, since the ozone acts at the bottom of said objects (23) in the form of receptacles where disinfection is most difficult, when degradation has not yet begun.

There can be intercalated with the outlets (27) intended for securing the objects (23) to be disinfected in the form of receptacles other outlets (27) for objects (23) having other characteristics, such as baby bottle nipples, for example. In any case, disinfection can be applied for any type of objects (23), and as regards receptacle-type objects (23), it can be applied for receptacles of any type in the form of flasks, bottles or the like.

The operating equipment housed in the body (1), whereby the ozone intended for disinfecting the objects (23) in the container (2) is produced is connected with a power supply and control system, from a connector socket (30) provided with a built-in fuse which is arranged in the outer front part of the body (1), as shown in Figure 11.

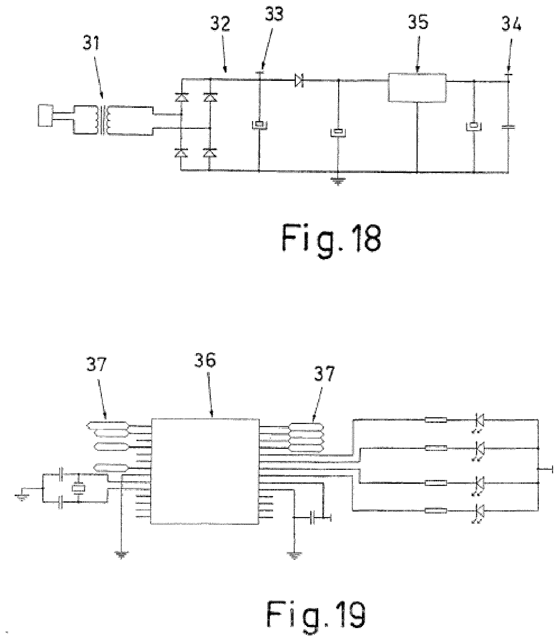

The power system comprises a power supply source (see Figure 18) which includes a transformer (31) and a rectifier bridge (32), providing a power outlet (33) for the connection of the elements of the operating equipment and a low-voltage outlet (34), through a stabilizer (35), for the connection of a microswitch (36) for operation control, through which signaling indicating the operating states and the behavior of the elements or parts of the functional unit is established.

The microswitch (36) is integrated in an electrical unit as depicted in Figure 19, with different ports (37) for being linked with activity signaling in relation to appliance operation.

In that sense, there are placed in the upper part of the body (1) a series of light indicators which show different operating states by means of turning on and off, a blinking function being provided, whereby the corresponding indicators show an error state of the corresponding functional elements or parts.

In that sense, there are envisaged an indicator indicating the connection of the appliance to a power line, an indicator indicating the activated or deactivated state of the air-driving motor pump (21), an indicator indicating the activated or deactivated state of the ozone generators (22), an indicator indicating the activation or deactivation of the system by means of a push-button switch, an indicator indicating a provisional connected but non-operating state, an indicator indicating the presence of air and ozone in the circulation conduit circulating towards the discharge outlets (27), and an indicator indicating the opening or closing of the mentioned conduit by means of the solenoid valve (not depicted).

Therefore, when the appliance is disconnected from the power supply line all the indicators are turned off, and when it is connected to a power supply line, the line indicator is turned on, all the other indicators still being off, in what can be called a rest state.

If the activation switch of the system is pressed on after establishing the connection to a power grid, the elements of the operating equipment are electrically powered and the system starts operating, all the indicators being turned on except the indicator indicating the provisional connected but non-operating state.

When the operation is activated by an established program which is controlled by the microswitch (36), the motor pump (21) starts operating first, driving air, and after a predetermined time the ozone generator or generators (22) start operating, so ozone production starts when there is already air circulating through the ozone generators (22), ozone production in vacuum which would cause degradation thus being prevented.

There is placed in the outlet conduit from the ozone generators (22) a detector (not depicted) capable of detecting the presence of air and ozone in the conduit, such that when said presence is evident, the mentioned detector (not depicted) activates the solenoid valve (not depicted) for opening the conduit, so circulation towards the outlets (27) where the objects (23) to be disinfected are arranged is only allowed when ozonated air already circulates, these circumstances being indicated by the corresponding indicator, suitable disinfection of the objects (23) thus being assured since the circulation conduit for circulation to the outlets (27) only opens when ozonated air is discharged therein.

In the operating state, air is driven and ozone is produced for a specific time, said operation being stopped automatically for prefixed time periods from time to time during operation, such that in said automatic stops the system is activated but non-operating in a provisional standby state in order to operate again every time the prefixed stop and shutdown time lapses when the established operation time lapses. Said operation and shutdown times can be regulated to adapt the function of the appliance according to the intended objects (23) of application.

Hence, ozone production and delivery during operation times saturates the liquid medium contained in the container (2) where the objects (23) to be disinfected are placed, whereas during the times of provisional standby state the disinfecting capacity of the medium gradually decreases until another operation time starts in which the medium is again saturated, active times and inactive times relating to operation thus taking place successively, during which times the disinfecting function on the objects (23) of application is continuous.

The power supply of the ozone generators (22) is established by means of a voltage doubler (not depicted) producing a very high application voltage (16,000 DC volts), for operation of the ultraviolet lamps producing the ozone.

The voltage doubler comprises a series of several successive amplifying stages, whereby the voltage is increased up to 16,000 volts for the operation, said doubler being provided with high impedance resistances at the outlet, whereby a rapid discharge of the voltage electric is achieved when outage occurs during operation to prevent discharges that may harm users when manipulating the appliance.

A hidden switch is envisaged in the closure between the coupling parts of the appliance that can be disassembled, particularly the coupling of the lid (3), which secures the assembly of the unit, through which switch the power supply to the operating equipment is disconnected, deactivating the entire system, whereas assembly coupling has not been completely established between the parts, users thus being prevented from being able to manipulate the inside of the appliance with a risk of accidents.

The power supply to the functional unit is also established by means of a system providing a square wave having constant pulses with variable gaps which can be regulated such that they allow determining automatic activation and deactivation in operating cycles perfectly determined for ozone production, while the pulse intensity can be regulated for adapting the revolutions of the motor pump (21) without altering electrical frequency. Operation is achieved under these conditions without magnetic influences, which allows applying the appliance of application in a domestic environment within the context of the guidelines established to that end.

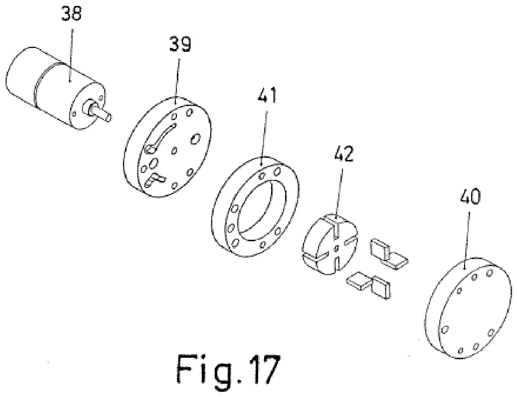

As depicted in Figure 17, the motor pump (21) comprises a motor (38) and a pump unit formed by respective lids (39 and 40) between which a ring (41) housing therein a rotor (42) is included. The component parts of the mentioned pump unit are graphite-injected plastics which allow self-lubrication suitable for operating at high revolutions with minimum wear, a very high driving capacity for discharging air during the operation of the appliance being achieved with a very small unit.

The component parts of the unit of the appliance that can be disassembled determine a modularity that allows prearrangement depending on the objects (23) for which the application is intended, such that the container (2) can have different shapes and dimensions in relation to said purpose, the arrangement of the coupling of the unit that can be disassembled being maintained.

## Claims

1. A disinfecting appliance comprising a operating unit with means (22) for producing ozone, said operating unit being adapted for air-driven delivery of said ozone to a container (2) adapted to receive the objects to be disinfected submerged in a liquid medium, wherein said disinfecting appliance comprises an assembly of three independent parts which are coupled together in an arrangement that can be disassembled, comprising:
a body (1) housing said operating unit,
said container (2), for the inclusion of objects to be disinfected in a liquid medium,
and a lid (3), closing the container (2), wherein said lid (3) incorporates means for locking said assembly in an integral unit and actuation means for releasing said assembly, wherein the body (1) incorporates, fixed to the front thereof, a first plate (4), whereas a second plate (7) is fixed to the lid (3), said plates (4 and 7) determining between them guided fitting formations for coupling of the assembly, while the container (2) is in turn incorporated in guided fitting coupling to the second plate (7) that is attached to the lid (3),
wherein the plates (4, 7) determine a tubular socket coupling (24), one of the parts of which is incorporated in the first plate (4) is connected with a first conduit coming from the operating unit for circulating air with ozone for disinfection, whereas another part of said tubular socket coupling (24) is attached to a second conduit projecting through the second plate (7) in several discharge outlets (27), and
wherein said discharge outlets (27) are downwardly inclined, some of the discharge outlets (27) incorporating tubular prolongations (28) with elements (29) having elastic radial arms for the arrangement of objects (23) to be disinfected.

2. The disinfecting appliance according to claim 1, wherein the means for locking the assembly comprise two latches (11 and 12) for locking the lid (3) and the container (2) and a latch (13) for locking the lid (3) and the body (1), said latches being linked by means of respective sets of rocker arms and connecting rods, with two operating push-buttons (14 and 15) in an arrangement whereby the latch (13) is individually actuated by means of one of said buttons (15), whereas the three latches (11, 12 and 13) are actuated at the same time with the other push-button (14).

3. The disinfecting appliance according to claim 1, wherein the operating unit comprises a motor pump (21) driving air and one or more ozone generators (22), through which the motor pump (21) drives the air, there then being an ozonated air circulation conduit in which there is arranged an on-off solenoid valve which is controlled by a detector which detects the presence of circulating air and ozone in the conduit.

4. The disinfecting appliance according to claims 1 and 3, wherein the power supply to the operating unit is established by means of a system comprising a power supply source with current transformation and rectification means, which provides a power outlet (33) for the connection of the elements of the operating equipment and a low-voltage outlet (34) through a stabilizer (35) for the connection of a microswitch (36) for operation control, through which signaling indicating the operating states is established.

5. The disinfecting appliance according to claims 3 and 4, wherein the power supply to the ozone generators (22) is established through a voltage doubler formed by multiple successive amplifying stages, providing a direct current voltage of 16,000 volts, high impedance resistances being placed at the outlet of said voltage doubler for rapid voltage discharge from the circuit during deactivation outage periods.

6. The disinfecting appliance according to claims 1 and 4, wherein in relation to the couplings for assembling the parts of the unit that can be disassembled there is incorporated a hidden switch, whereby the electrical connection to the functional unit is interrupted while the parts of the unit that can be disassembled are not perfectly coupled together.

7. The disinfecting appliance according to claim 3, wherein the motor pump (21) comprises a pump unit formed by graphite-injected plastic elements, determining the self-lubrication of said pump unit during operation.

## Patentansprüche

1. Desinfektionsgerät, umfassend eine Betriebseinheit mit einem Mittel (22) zum Erzeugen von Ozon, wobei die Betriebseinheit angepasst ist für die luftgetriebene Abgabe von Ozon an einen Container (2), der angepasst ist, um die zu desinfizierenden Gegenstände aufzunehmen, die in einem flüssigen Medium eingetaucht sind,
wobei das Desinfektionsgerät eine Baugruppe aus drei unabhängigen Teilen umfasst, die in einer Anordnung so untereinander verbunden sind, dass sie zerlegt werden kann, umfassend:
ein Gehäuse (1), in dem die Betriebseinheit untergebracht ist,
den Container (2) für die Aufnahme von zu desinfizierenden, in ein flüssiges Medium Gegenständen,
und einen Deckel (3), der den Container (2) verschließt, wobei der Deckel (3) Mittel zum Verschließen der Baugruppe in einer Gesamteinheit und Stellmittel für die Freigabe der Baugruppe integriert, wobei das Gehäuse (1) an seiner Vorderseite befestigt eine erste Platte (4) integriert, während eine zweite Platte (7) am Deckel (3) befestigt ist, wobei die Platten (4 und 7) zwischen ihnen geführte Einpassformungen zum Ankoppeln der Baugruppe festlegen, während der Container (2) wiederum in die geführte Einpassformung zur zweiten Platte (7) integriert ist, die am Deckel (3) befestigt ist,
wobei die Platten (4, 7) eine rohrförmige Muffenkupplung (24) festlegen, von der eines der Teile, das in die erste Platte (4) integriert ist, mit einer ersten Leitung verbunden ist, die aus der Betriebseinheit kommt, um Luft mit Ozon zur Desinfektion zu zirkulieren, während ein anderes Teil der rohrförmigen Muffenkupplung (24) an einer zweiten Leitung befestigt ist, die durch die zweite Platte (7) in verschiedenen Abgabeöffnungen (27) projiziert, und
wobei die Abgabeöffnungen (27) nach unten geneigt sind, einige der Abgaböffnungen (27) rohrförmige Verlängerungen (28) mit Elementen (29) integrieren, die elastische radiale Arme für die Anordnung der zu desinfizierenden Gegenstände (23) besitzen.

2. Desinfektionsgerät nach Anspruch 1, wobei die Mittel zum Verschließen der Baugruppe zwei Sperrriegel (11 und 12) zum Verschließen des Deckels (3) und des Containers (2) sowie einen Sperrriegel (13) zum Verschließen des Deckels (3) und des Gehäuses (1) umfassen, wobei die Sperrriegel über jeweilige Kipphebel- und Verbindungsstangensets angeschlossen sind, mit zwei Betätigungsdruckknöpfen (14 und 15) in einer Anordnung, wodurch der Sperrriegel (13) über einer der Knöpfe (15) einzeln betätigt wird, während die drei Sperrriegel (11, 12 und 13) gleichzeitig mit dem anderen Druckknopf (14) betätigt werden.

3. Desinfektionsgerät nach Anspruch 1, wobei die Betriebseinheit eine Motorpumpe (21) umfasst, die Luft- und einen oder mehrere Ozongeneratoren (22) antreibt, durch die die Motorpumpe (21) die Luft antreibt, des Weiteren eine Leitung für die Zirkulation von mit Ozon versetzter Luft vorhanden ist, in der ein Ein-Aus-Magnetventil angeordnet ist, das von einem Detektor gesteuert wird, der die Anwesenheit von zirkulierender Luft und Ozon in der Leitung erkennt.

4. Desinfektionsgerät nach Ansprüchen 1 und 3, wobei die Stromversorgung der Betriebseinheit über ein System hergestellt wird, das eine Stromversorgungsquelle mit einem Mittel zu Stromübersetzung und -gleichrichtung umfasst, das eine Leistungsabgabe (33) für die Verbindung der Elemente des Betriebsgeräts und eine Abgabe mit niedriger Spannung (34) durch einen Konstanthalter (35) für die Verbindung eines Mikroschalters (36) zur Betriebssteuerung, durch die die Signalisierung anzeigt, dass der Betriebszustand hergestellt ist, bereitstellt.

5. Desinfektionsgerät nach Ansprüchen 3 und 4, wobei die Stromversorgung des Ozongenerators (22) durch einen Spannungsverdoppler hergestellt wird, der aus mehreren aufeinanderfolgenden Verstärkungsstufen gebildet ist und eine Gleichspannung von 16 000 Volt bereitstellt, wobei zur schnellen Spannungsentladung aus dem Schaltkreis während der Deaktivierungsstillstandzeiten Widerstände mit hoher Impedanz am Ausgang des Spannungsverdopplers platziert sind.

6. Desinfektionsgerät nach Ansprüchen 1 und 4, wobei in Bezug auf die Verkupplungen zum Anbau der Teile der Einheit, die abgebaut werden können, ein verborgener Schalter integriert ist, wodurch die elektrische Verbindung der Funktionseinheit unterbrochen wird, solange die Teile der Einheit, die abgebaut werden können, nicht einwandfrei untereinander verkoppelt sind.

7. Desinfektionsgerät nach Anspruch 3, wobei die Motorpumpe (21) eine Pumpeinheit umfasst, die aus Kunststoffelementen mit eingespritztem Graphite gebildet ist, die die Selbstschmierung der Pumpeneinheit während des Betriebs festlegen.

## Revendications

1. Appareil de désinfection comprenant une unité fonctionnelle avec un moyen (22) de production d'ozone, ladite unité fonctionnelle étant conçue pour la délivrance entraînée par l'air dudit ozone à un contenant (2) conçu pour recevoir les objets à désinfecter submergés dans un milieu liquide,
dans lequel ledit appareil de désinfection comprend un montage de trois parties indépendantes qui se couplent entre elles selon un agencement démontable, comprenant :
un corps (1) logeant ladite unité fonctionnelle,
ledit contenant (2), pour l'inclusion d'objets à désinfecter dans un milieu liquide,
et un couvercle (3), fermant ledit contenant (2), dans lequel ledit couvercle (3) incorpore un moyen de blocage dudit montage en une unité intégrale et un moyen d'actionnement pour libérer ledit montage, dans lequel le corps (1) incorpore, fixé à l'avant de celui-ci, une première plaque (4), alors qu'une seconde plaque (7) est fixée au couvercle (3), lesdites plaques (4 et 7) déterminant entre elles des formations d'encastrement guidé pour coupler le montage, alors que le contenant (2) est à son tour incorporé dans un couplage d'encastrement guidé avec la seconde plaque (7) qui est fixée au couvercle (3),
dans lequel les plaques (4, 7) déterminent un couplage de raccord tubulaire (24), dont une des parties, incorporée dans la première plaque (4), est raccordée à un premier conduit venant de l'unité fonctionnelle pour faire circuler l'air avec l'ozone pour la désinfection, alors que l'autre partie dudit couplage de raccord tubulaire (24) est fixée à un second conduit se projetant à travers la seconde plaque (7) en plusieurs sorties de décharge (27), et
dans lequel les sorties de décharge (27) sont inclinées vers le bas, une partie des sorties de décharge (27) incorporant des prolongations tubulaires (28) avec des éléments (29) ayant des bras radiaux élastiques pour l'agencement d'objets (23) à désinfecter.

2. Appareil de désinfection selon la revendication 1, dans lequel le moyen de blocage du montage comprend deux verrous (11 et 12) pour bloquer le couvercle (3) et le contenant (2) et un verrou (13) pour bloquer le couvercle (3) et le corps (1), lesdits verrous étant liés au moyen d'ensembles respectifs de balanciers et de bielles, avec deux boutons poussoirs fonctionnels (14 et 15) dans un agencement permettant que le verrou (13) soit individuellement actionné au moyen de l'un desdits boutons (15), alors que les trois verrous (11, 12 et 13) sont actionnés en même temps avec l'autre bouton poussoir (14).

3. Appareil de désinfection selon la revendication 1, dans lequel l'unité fonctionnelle comprend une motopompe (21) entraînant l'air et un ou plusieurs générateurs d'ozone (22), par lesquels la motopompe (21) entraîne l'air, après quoi se trouve un conduit de circulation d'air ozoné dans lequel est disposée une électrovanne marche-arrêt qui est commandée par un détecteur qui détecte la présence d'air circulant et d'ozone dans le conduit.

4. Appareil de désinfection selon les revendications 1 et 3, dans lequel l'alimentation électrique de l'unité fonctionnelle est établie au moyen d'un système comprenant une source d'alimentation électrique avec un moyen de transformation et de rectification de courant, qui fournit une prise de courant (33) pour le raccordement des éléments de l'équipement fonctionnel et une prise basse tension (34) dans un stabilisateur (35) pour le raccordement d'un microrupteur (36) pour la commande de fonctionnement, par laquelle une signalisation indiquant les états fonctionnels est établie.

5. Appareil de désinfection selon les revendications 3 et 4, dans lequel l'alimentation électrique des générateurs d'ozone (22) est établie par un doubleur de tension formé par de multiples étapes d'amplification successives, fournissant une tension de courant continu de l'ordre de 16 000 volts, des résistances à impédance élevée étant placées au niveau de la sortie dudit doubleur de tension pour la décharge rapide de tension du circuit durant les périodes de coupure de désactivation.

6. Appareil de désinfection selon les revendications 1 et 4, dans lequel relativement aux couplages pour le montage des parties de l'unité qui peuvent être démontées, un interrupteur encastré est incorporé, permettant d'interrompre le raccordement électrique à l'unité fonctionnelle lorsque les parties de l'unité qui peuvent être démontées ne sont pas parfaitement couplées entre elles.

7. Appareil de désinfection selon la revendication 3, dans lequel la motopompe (21) comprend une unité de pompe formée par des éléments en plastique injecté de graphite, déterminant l'autolubrification de ladite unité de pompe pendant le fonctionnement.
